# EUROPÄISCHE PATENTSCHRIFT

(11) **EP 2 818 137 B1**
(45) Veröffentlichungstag und Bekanntmachung des Hinweises auf die Patenterteilung: **28.06.2017**
(21) Anmeldenummer: 14166554.7
(22) Anmeldetag: 30.04.2014
(51) Int. Cl.: A61F 2/18

(54) **Gehörknöchelchenprothese mit CT- oder MRT-Marker**
Auditory ossicle prosthesis with CT or MRT markers
Prothèse d'osselet avec marqueur CT ou MRT

(30) Priorität: 24.06.2013 DE 202013102732 U
(43) Veröffentlichungstag der Anmeldung: 31.12.2014
(73) Patentinhaber: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE)
(74) Vertreter: Kohler Schmid Möbus Patentanwälte

(56) Entgegenhaltungen:
- DE-B3-102010 046 457
- US-A1- 2011 009 961

## Beschreibung

Die Erfindung betrifft eine Gehörknöchelchenprothese, die zum Ersatz oder Überbrücken mindestens eines Elements der menschlichen Gehörknöchelchenkette ausgebildet ist, mit einem länglichen, Schall übertragenden Prothesenkörper, welcher an seinem einen Ende ein erstes Ankoppelelement aufweist, das als eine Kopfplatte zur Anlage der Prothese am Trommelfell oder als ein Clip zur mechanischen Verbindung mit einem Glied der Gehörknöchelchenkette, insbesondere mit dem Ambossfortsatz oder mit dem Hammergriff, oder als ein Anschlussstück zur Schall leitenden Verbindung mit einem Aktor-Endstück eines aktiven Hör-Implantats gestaltet ist, wobei der Prothesenkörper an seinem anderen Ende ein zweites Ankoppelelement zur mechanischen Verbindung mit einem weiteren Glied oder Teilen eines Gliedes der Gehörknöchelchenkette oder direkt mit dem Innenohr aufweist.

Derartige Gehörknöchelchenprothesen sind beispielsweise bekannt aus DE 10 2007 062 151 B3 oder aus DE 10 2010 046 457 B3 oder auch aus DE 10 2005 034 103 B3.

Das menschliche Mittelohr mit seinen Gehörknöchelchen hat die Funktion, die über den äußeren Gehörgang auf das Trommelfell auftreffenden Schallwellen auf das mit Flüssigkeit gefüllte Innenohr zu übertragen. Die drei Gehörknöchelchen sind der am Trommelfell befestigte Hammer (lat. malleus), der Steigbügel (lat. stapes), der über seine Fußplatte (lat. basis stapedis) mit dem Innenohr verbunden ist, und der Amboss (lat. incus), der sich zwischen dem Hammer und dem Steigbügel befindet und mit diesen gelenkig verbunden ist. Beispielsweise die Otosklerose ist eine Erkrankung des menschlichen Felsenbeins (= Knochen, in dem das gesamte Ohr sitzt), bei der es durch entzündungsähnliche Knochenumbauprozesse zu einer Fixierung des normalerweise locker schwingenden Steigbügels kommen kann. Dadurch wird das Schallsignal nicht oder nur unvollkommen über die Gehörknöchelchenkette auf das Innenohr übertragen, was zur Schwerhörigkeit führt.

Gehörknöchelchenprothesen dienen der Verbesserung der Schallübertragung bei unterschiedlichen pathologischen Befunden. Sie werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchen-prothese weist dabei zwei Enden auf, wobei je nach den konkreten Gegebenheiten das eine Ende der Gehörknöchelchenprothese beispielsweise mittels einer Kopfplatte am Trommelfell oder am Aktor-Endstück eines aktiven Hörgeräts und das andere Ende der Gehörknöchelchenprothese beispielsweise am Steigbügel der menschlichen Gehörknöchelchenkette befestigt oder direkt ins Innenohr getaucht wird.

Drei besonders häufig verwendete Arten von Gehörknöchelchenprothesen sind die Steigbügel-Prothesen, die Partial-Prothesen und die Total-Prothesen. Steigbügel-Prothesen (=Stapes-Prothesen) werden am Amboss fixiert und ragen über einen Stempel (=Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Des Weiteren unterscheidet man generell zwischen *passiven* Gehörknöchelchenprothesen und *aktiven* Hör-Implantaten:

Die eingangs zitierten Patente DE 10 2007 062 151 B3 und DE 10 2005 034 103 B3 beschreiben jeweils passive Gehörknöchelchenprothesen, die nach ihrer Implantation im Mittelohr eine verbesserte Schallleitung zum Innenohr aufgrund ihres geometrischen Aufbaus gänzlich ohne Zuhilfenahme elektronischer Verstärker bewirken.

Demgegenüber verwenden aktive Hör-Implantate elektronische Verstärkungselemente, welche in der Regel außerhalb des Ohres angeordnet sind. Diese übertragen aktiv elektronisch bearbeitete Schallsignale über ein Aktor-Endstück unter Umgehung des Trommelfells direkt ins Mittelohr, wo der Schall dann entweder auf die natürliche Gehörknöchelchenkette geleitet wird oder wiederum auf ein Ankoppelelement einer Gehörknöchelchenprothese. Eine Anordnung zum Einstellen und Fixieren der Relativlage zwischen einerseits einem Aktor-Endstück eines aktiven Hör-Implantats und andererseits einem Glied der Gehörknöchelchenkette oder einem Ankoppelteil zum menschlichen Innenohr ist etwa bekannt aus eingangs zitierten deutschen Patent DE 10 2010 046 457 B3.

Alle genannten Gehörknöchelchenprothese und Hör-Implantate - seien sie nun aktiv oder passiv - sind nach Abschluss der Implantation und insbesondere nach dem Verheilen der Operationswunde an ihren im Mittelohr eingeschlossenen Teilen prinzipiell nicht mehr von außen zugänglich. Ergeben sich dann jedoch Komplikationen oder Fehlfunktionen, insbesondere eine ungenügende Signalübertragung - beispielsweise aufgrund von Verwachsungen während des Einheil-Prozesses und daraus resultierende Lage-Verschiebungen der implantierten Anordnung - muss das Mittelohr wiederum durch einen chirurgischen Eingriff geöffnet werden, so dass überhaupt erst festgestellt werden kann, in welcher räumlichen Lage sich die Prothesenteile relativ zur Mittelohr-Umgebung befinden und ob das aufgetretene Problem damit in einem Zusammenhang steht. Beim Eröffnen des Mittelohres besteht die große Gefahr, dass die Qualität der Ankopplung nicht geprüft werden kann, weil nämlich das Implantat andererseits auch bereits am Trommelfell angekoppelt war, bedingt durch die Technik einer neuerlichen Operation jedoch genau hier der Zugang gewählt werden muss. In anderen Worten, die Ankopplung, welche man prüfen will, wird in einem vorgelagerten OP-Schritt schon derart stark beeinflusst, dass der Chirurg am Ende oft nur erahnen kann, was der eigentliche Fehler war. Die Patentschrift DE 10 2010 046 457 B3 offenbart eine Gehörknöchelchenprothese gemäß dem Oberbegriff von Anspruch 1.

Aufgabe der vorliegenden Erfindung ist es demgegenüber, eine gattungsgemäße passive oder aktive Gehörknöchelchenprothese der eingangs beschriebenen Art mit möglichst simplen technischen Mitteln und wirtschaftlich preisgünstig dahin gehend zu verbessern, dass dem behandelnden Arzt nach einer Implantation der Prothese auch ohne für den Patienten belastende erneute chirurgische Eingriffe die Möglichkeit einer einfach durchführbaren Kontrolle der exakten räumlichen Position des Implantats im Mittelohr des Patienten eröffnet wird.

Erfindungsgemäß wird diese Aufgabe auf ebenso überraschend einfache wie wirkungsvolle Art und Weise dadurch gelöst, dass das erste Ankoppelelement und/oder das zweite Ankoppelelement und/oder der Prothesenkörper eine Markierungseinrichtung aufweist, die mindestens zwei voneinander beabstandete, erhabene, an einer Außenfläche des ersten Ankoppelelements und/oder des zweiten Ankoppelelements und/oder des Prothesenkörpers angeordnete Markierungskörper umfasst, und dass die Markierungskörper so gestaltet sind, dass sie post-operativ in einem im Mittelohr eines Patienten implantierten Zustand der Gehörknöchelchenprothese auf MR (=Magnetresonanz)-Aufnahmen oder auf CT (=Computer-Tomographie)-Aufnahmen getrennt voneinander deutlich erkennbar und aufgrund ihrer geometrischen Ausgestaltungen und/oder aufgrund ihrer Materialeigenschaften eindeutig zuordenbar sind und durch ihre vorbekannten relativen Positionen in oder an der Gehörknöchelchenprothese eine genaue Erkennung und Bestimmung der exakten räumlichen Lage der Gehörknöchelchenprothese im Mittelohr des Patienten ermöglichen.

Auf diese Weise kann erreicht werden, dass die Lage des Implantats und damit die Ankopplung an die menschliche Gehörknöchelchenkette ohne einen chirurgischen Eingriff und damit ohne mechanischen Einfluss, also quasi berührungslos beurteilt werden kann.

Besonders bevorzugt sind Ausführungsformen der erfindungsgemäßen Gehörknöchelchenprothese, die sich dadurch auszeichnen, dass mindestens einer der Markierungskörper magnetisches Material enthält, welches bei einer MRI-Aufnahme eine markante Störung des äußerst homogenen NMR-Magnetfelds und damit in der Bildgebung entsprechend markante und gut erkennbare Objekt-Signale erzeugt.

Noch günstiger sind insbesondere für NMR-Anwendungen Ausführungsformen der Erfindung, bei denen mindestens einer der Markierungskörper kohlenwasserstoffhaltiges Kunststoffmaterial wie etwa Teflon enthält. Dieses stört die MRI-Aufnahme nicht, so dass auch sehr kleine und feine Strukturen mit dieser Technik verzerrungsfrei aufgelöst werden können, wobei die in dem Material enthaltenen Protonen ein eigenes NMR-Echosignal aussenden, welches die charakteristische Form des jeweiligen Markierungskörpers sowie seine exakte Lage besonders gut wiedergibt.

Vorteilhaft sind auch Ausführungsformen der erfindungsgemäßen Gehörknöchelchenprothese, bei welchen mindestens einer der erhabenen Markierungskörper eine rundliche, vorzugsweise eine kugelige oder tropfenförmige Form aufweist.

Bei anderen Ausführungsformen weist mindestens einer der erhabenen Markierungskörper eine spitze und/oder eckige Form, insbesondere Kegel- oder Würfelform auf, so dass bei entsprechender Auflösung die räumlichen Richtungen deutlich und klar auf der Aufnahme zu erkennen sind.

Besonders bevorzugt ist eine Klasse von Ausführungsformen der Erfindung, bei denen mindestens drei der erhabenen Markierungskörper gleichmäßig azimutal um die Längsachse des Prothesenkörpers angeordnet sind, so dass Darstellungen im Raum gut beurteilt werden können.

Eine vorteilhafte Weiterbildung dieser Klasse von Ausführungsformen zeichnet sich dadurch aus, dass das erste Ankoppelelement als runde diskusförmige Kopfplatte zur Anlage am Trommelfell ausgebildet ist, und dass die erhabenen Markierungskörper am Außenumfang der Kopfplatte und/oder an deren dem Trommelfell im implantierten Zustand der Gehörknöchelchenprothese abgewandten Unterseite positioniert sind.

Bei einer weiteren Klasse von Ausführungsformen der erfindungsgemäßen Gehörknöchelchenprothese umfasst die Markierungseinrichtung zusätzlich mindestens einen Hohlraum, eine Bohrung oder eine napfartige Einbuchtung am oder im ersten Ankoppelelement und/oder zweiten Ankoppelelement und/oder am Prothesenkörper.

Eine vorteilhafte Weiterbildung in dieser Klasse von Ausführungsformen zeichnet sich dadurch aus, dass der Hohlraum, die Bohrung oder die Einbuchtung mit magnetischem Material und/oder mit kohlenwasserstoffhaltigem Kunststoffmaterial eingefasst oder ausgekleidet ist.

Diese Ausführungsformen lassen dadurch noch weiter verbessern, dass die Markierungseinrichtung mehrere - vorzugsweise mindestens drei - Hohlräume, Bohrungen oder napfartige Einbuchtungen umfasst, die gleichmäßig azimutal um die Längsachse des Prothesenkörpers angeordnet sind.

Besonders bevorzugte Ausführungsformen der erfindungsgemäßen Gehörknöchelchenprothese sind dadurch gekennzeichnet, dass der Prothesenkörper mindestens ein Gelenk, insbesondere ein Kugelgelenk aufweist. Dies ist vorteilhaft im Hinblick auf eine besonders hohe postoperative Beweglichkeit der Prothese. Möglich sind auch Weiterbildungen, bei denen eine Vielzahl von aneinander angrenzenden weiteren Drehelementen vorgesehen ist, vorzugsweise eine Kugelgelenkkette. Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse post-operative Mobilität aufweist, so dass sich die Kopfplatte postoperativ selbstständig der Position des Trommelfells angleichen kann. Da zudem die anatomischen Gegebenheiten des Ohrs, wie beispielsweise die Lage, die Form und die Größe des Steigbügel, des Ambosses, des Hammers und des Trommelfells variieren, ist es sehr vorteilhaft, wenn Gehörknöcheichenprothesen nicht starr ausgebildet sind, sondern eine gewisse Flexibilität oder Variabilität aufweisen.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein.

In vielen Ausführungsformen der Erfindung wird das erste Ankoppelelement eine zur Anlage am Trommelfell ausgebildete Kopfplatte umfassen. Bei anderen Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäßen Gehörknöchelchenprothese zeichnet sich dadurch aus, dass das zweite Ankoppelelement als Platte, als Hülse, als Schlinge, als geschlossene Glocke, als einfach oder mehrfach geschlitzte Glocke, als Stempel oder als Clip zur mechanischen Verbindung mit einem weiteren Glied der Gehörknöchelchenkette ausgebildet ist.

Bei Weiterbildungen dieser Ausführungsformen ist die Prothese über die Kopfplatte einerseits am Trommelfell und über das zweite Befestigungselement andererseits am Amboss oder am Steigbügel befestigt.

Alternative Ausgestaltungen können vorsehen, dass die Gehörknöchelchenprothese an ihrem das zweite Ankoppelelement tragenden Ende mittels Perforation der Steigbügelfiußplatte (=Stapedektomie bzw. Stapedotomie) und/oder mittels Eröffnung der menschlichen Hörschnecke (=Cochleotomie) direkt an das Innenohr angekoppelt ist, insbesondere über ein als Kolben (=Piston) ausgeführtes zweites Ankoppelelement.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfireie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Ein als Kopfplatte ausgeführtes erstes Ankoppelelement der erfindungsgemäßen Gehörknöchelchenprothese sollte grundsätzlich eine wachstumsfördernde Beschichtung, ein direkt ins Innenohr führendes, etwa in Form eines Kolbens ausgebildetes zweites Ankoppelelement hingegen eine wachstumshemmende Beschichtung aufweisen.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Möglich sind aber auch Ausführungsformen der Erfindung, bei denen die gesamte Prothese oder Teile davon aus biokompatiblen Kunststoffen, insbesondere Silikon, Polytetrafluorethylen (PTFE) oder Faserverbundwerkstoffen hergestellt sind. Mit diesen Materialien können post-operative Abstoßungsreaktionen in den meisten Fällen ebenfalls verhindert werden.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestalteten Gehörknöchelchenprothese erreichen.

Ein solches "mechanisches Tuning" kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Nützlich zur Anwendung der vorliegenden Erfindung ist ein Verfahren zur post-operativen Erkennung und exakten Bestimmung der räumlichen Lage einer mit einer Markierungseinrichtung der oben beschriebenen Art ausgestatteten Gehörknöchelchenprothese im Mittelohr eines Patienten im implantierten Zustand relativ zur Mittelohr-Umgebung der Prothese. Dieses Verfahren zeichnet sich dadurch aus, dass nach der Implantation der Gehörknöchelchenprothese ins Mittelohr MR-Bilder oder CT-Bilder vom Mittelohr-Bereich des Patienten aufgenommen werden, und dass die exakte relative Lage der Gehörknöchelchenprothese in Bezug auf ihre Umgebung im Mittelohr aufgrund der in den Aufnahmen erkennbaren, eindeutig zuordenbaren geometrischen Ausgestaltung und/oder Materialeigenschaften der Markierungskörper der Markierungseinrichtung sowie aufgrund der vorbekannten relativen Position der Markierungseinrichtung in oder an der Gehörknöchelchenprothese ermittelt wird.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können jeweils einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1: eine schematische räumliche Darstellung einer ersten Ausführungsform der erfindungsgemäßen Gehörknöchelchenprothese mit einer Trommelfell-Kopfplatte als erstem Ankoppelelement und einem stempelförmigen zweiten Ankoppelelement zur Auflage auf der SteigbügelFußplatte sowie mit kugelförmigen, um den Außenumfang der Kopfplatte angeordneten Markierungskörpern;
- Fig. 2: eine Ausführungsform wie in Fig. 1, aber mit unterhalb der Kopfplatte angeordneten halbkugelförmigen Markierungskörpern;
- Fig. 3: eine Ausführungsform mit einer Trommelfell-Kopfplatte als erstem und einer geschlitzten Glocke zur Befestigung am Steigbügel als zweitem Ankoppelelement sowie mit kugeligen Markierungskörpern auf der Glocke und napfförmigen Einbuchtungen in der Kopfplatte;
- Fig. 4: eine Ausführungsform mit einem Clip als erstem Ankoppelelement und einem Piston zum Eintauchen ins Innenohr als zweitem Ankoppelelement, sowie mit einer zusätzlichen Bohrung im Mittelteil des Prothesenkörpers; und
- Fig. 5: eine Ausführungsform mit einem Anschlussstück zur Schall leitenden Verbindung mit einem Aktor-Endstück eines aktiven Hör-Implantats.

Die in den Figuren der Zeichnung schematisch dargestellten, im Detail unterschiedlich gestalteten Ausführungsformen der erfindungsgemäßen **Gehörknöchelchenprothese 10; 20; 30; 40; 50** weisen jeweils einen schaftförmigen länglichen, Schall übertragenden **Prothesenkörper 13; 23; 33; 43; 53** auf, welcher am einen Ende jeweils ein **erstes Ankoppelelement 11; 21; 31; 41; 51** trägt, das als eine **Kopfplatte 11; 21; 31** zur Anlage der Prothese am Trommelfell oder als ein **Clip 41** zur mechanischen Verbindung mit einem Glied der Gehörknöchelchenkette, insbesondere mit dem Ambossfortsatz oder mit dem Hammergriff dient, oder als ein **Anschlussstück 51** zur Schall leitenden Verbindung mit einem **Aktor-Endstück 59** eines aktiven Hör-Implantats gestaltet ist. Am anderen Ende des Prothesenkörpers 13; 23; 33; 43; 53 sitzt ein **zweites Ankoppelelement 12; 22; 32; 42; 52,** weiches in vielfältigen geometrischen Formen zur mechanischen Verbindung mit einem weiteren Glied oder Teilen eines Gliedes der Gehörknöchelchenkette als Platte, als Hülse, als Schlinge, als **geschlossene Glocke 12; 22,** als einfach oder **mehrfach geschlitzte Glocke 32,** als **Stempel 52** oder als Clip oder als **Piston 42** zum direkten Eintauchen ins Innenohr gestaltet sein kann.

Erfindungsgemäß ist die Gehörknöchelchenprothese 10; 20; 30; 40; 50 dadurch gekennzeichnet, dass das erste Ankoppelelement 11; 21; 41; 51 und/oder das zweite Ankoppelelement 32; 52 und/oder der Prothesenkörper 43 eine Markierungseinrichtung aufweist, die mindestens zwei voneinander beabstandete, erhabene, an einer Außenfläche des ersten Ankoppelelements 11; 21; 41; 51 und/oder des zweiten Ankoppelelements 32; 52 und/oder des Prothesenkörpers 43 angeordnete **Markierungskörper 14; 24; 34"; 44'; 54a', 54b', 54"** umfasst, und dass die Markierungskörper 14; 24; 34"; 44'; 54a', 54b', 54" so gestaltet sind, dass sie post-operativ in einem im Mittelohr eines Patienten implantierten Zustand der Gehörknöchelchenprothese 10; 20; 30; 40; 50 auf MR-Aufnahmen oder auf CT-Aufnahmen getrennt voneinander deutlich erkennbar und aufgrund ihrer geometrischen Ausgestaltungen und/oder aufgrund ihrer Materialeigenschaften eindeutig zuordenbar sind und durch ihre vorbekannten relativen Positionen in oder an der Gehörknöchelchenprothese 10; 20; 30; 40; 50 eine genaue Erkennung und Bestimmung der exakten räumlichen Lage der Gehörknöchelchenprothese 10; 20; 30; 40; 50 im Mittelohr des Patienten ermöglichen.

Der Markierungskörper 14; 24; 34"; 44'; 54a', 54b', 54" kann magnetisches Material, aber beispielsweise auch Kohlenwasserstoffhaltiges Kunststoffmaterial enthalten, um eine besonders gute Erkennbarkeit in den MR- oder CT-Aufnahmen zu gewährleisten.

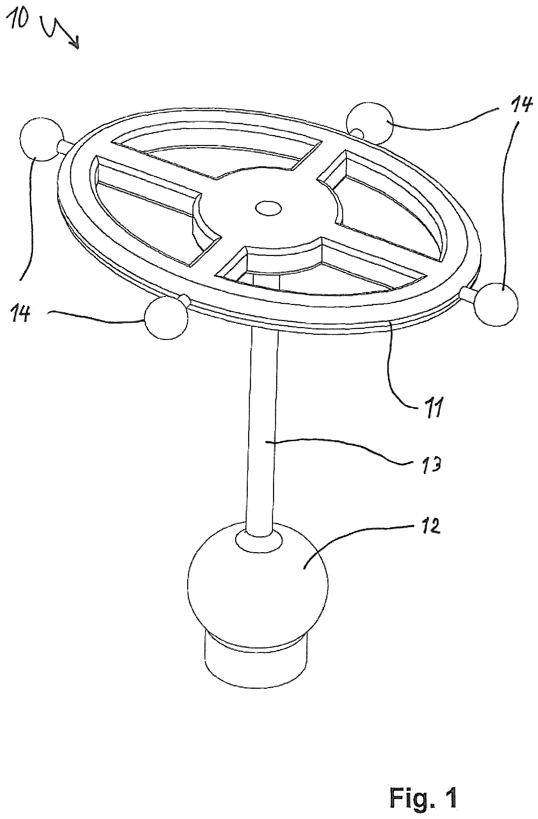

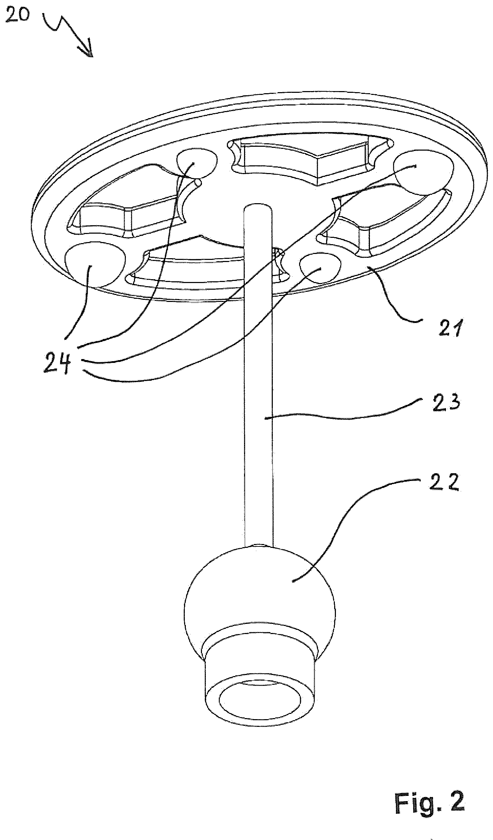

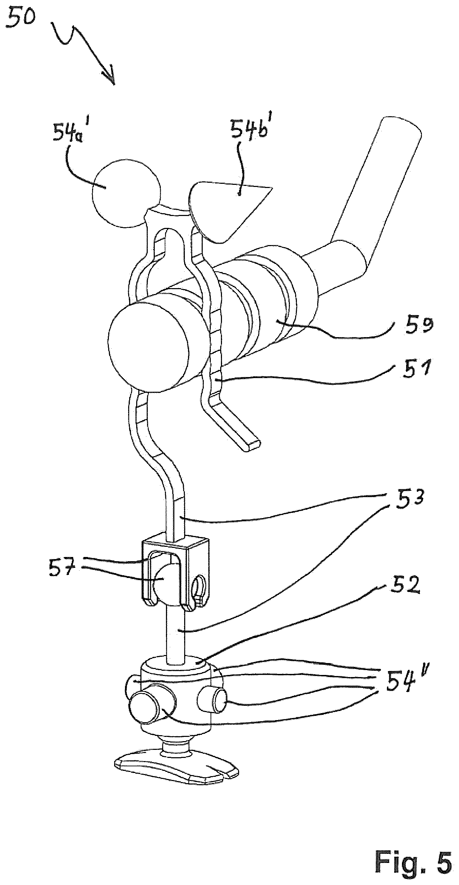

Auch die geometrischen Formen der Markierungskörper 14; 24; 34"; 44'; 54a', 54b', 54" können individuell und höchst unterschiedlich gestaltet sein: Bei den in den Figuren 1 bis 4 dargestellten Ausführungsformen weisen die Markierungskörper 14; 24; 34"; 44' rundliche, teilweise kugelige oder halbkugelförmige Formen auf. In Fig. 5 ist auch eine Ausführungsform der erfindungsgemäßen Gehörknöchelchenprothese 50 gezeigt, bei welcher die der Markierungskörper 54a', 54b', 54" eine spitze und/oder eckige Form, insbesondere Kegel- oder Würfelform aufweisen.

Wie bei den Ausführungsformen der Figuren 1 bis 3 und 5 dargestellt, kann es für die Funktion günstig sein, wenn mehrere - hier drei oder vier - der erhabenen Markierungskörper 14; 24; 34"; 54" gleichmäßig azimutal um die Längsachse des Prothesenkörpers 13; 23; 33; 53 angeordnet sind.

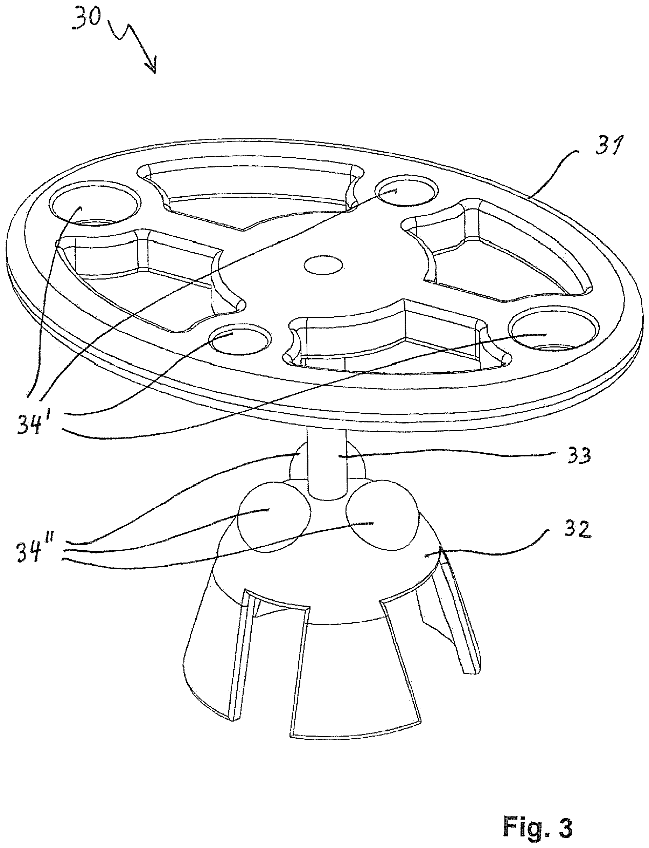

Die Gehörknöchelchenprothese 10; 20; 30 besitzt als erstes Ankoppelelement 11; 21; 31 jeweils eine runde diskusförmige Kopfplatte, die zur Anlage am Trommelfell ausgebildet ist. In den beiden ersten dargestellten Ausführungsformen sind die erhabenen Markierungskörper 14; 24' am Außenumfang der Kopfplatte beziehungsweise an deren dem Trommelfell im implantierten Zustand der Gehörknöchelchenprothese 10; 20 abgewandten Unterseite positioniert, bei der Gehörknöchelchenprothese 30 in Fig. 3 auf der Oberseite des als mehrfach geschlitzte Glocke ausgeführten zweiten Ankoppelelements 32.

Bei der letztgenannten Prothese gemäß Fig. 3 umfasst die Markierungseinrichtung zusätzlich noch vier **napfartige Einbuchtungen 34'** auf der Trommelfell-zugewandten Oberseite des als Kopfplatte ausgebildeten ersten Ankoppelelements 31, welche gleichmäßig azimutal um die Längsachse des Prothesenkörpers 33 verteilt angeordnet sind.

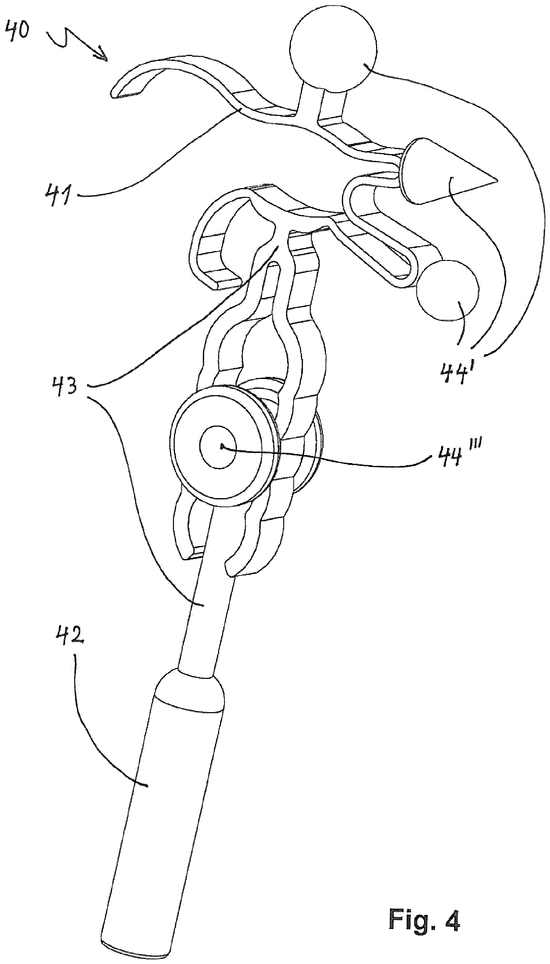

Die Gehörknöchelchenprothese 40 in Fig. 4 weist am Prothesenkörper 43 zusätzlich eine **Bohrung 44'''** auf, die ebenfalls zur Lokalisierung der exakten Lage der Prothese auf MR- oder CT-Aufnahmen dient. In der Zeichnung nicht eigens dargestellte weitere Ausführungsformen der erfindungsgemäßen Gehörknöchelchenprothese können zu diesem Zweck neben den gezeigten Einbuchtungen oder Bohrungen auch entsprechende Hohlräume am oder im ersten Ankoppelelement und/oder zweiten Ankoppelelement und/oder am Prothesenkörper umfassen. Diese Hohlräume, Bohrungen oder Einbuchtungen können zur besseren Erkennbarkeit auf den Aufnahmen auch mit magnetischem Material und/oder mit kohlenwasserstoffhaltigem Kunststoffmaterial eingefasst oder ausgekleidet sein.

In der Ausführungsform nach Fig. 5 ist in den Prothesenkörper 53 ein **Kugelgelenk 57** integriert, um die Beweglichkeit der Gehörknöchelchenprothese 50 zwischen ihren Ankoppelelementen 51, 52 zu erhöhen, wodurch eine gewisse postoperative Flexibilität der Prothese erreicht wird.

Die Massenverteilung der einzelnen Teile einer erfindungsgemäßen Gehörknöchelchenprothese kann in Abhängigkeit von einem gewünschten, vorgebbaren Frequenzgang der Schallleitung im Mittelohr derart berechnet sein, dass ein individuelles akustisches Tuning der Schallleitungs-Eigenschaften ermöglicht wird. Dies kann auch - bei in der Zeichnung nicht dargestellten Ausführungsformen der Erfindung - durch an die Gehörknöchelchenprothese anclipsbare "Trimm-Massen" erreicht werden.

## Patentansprüche

1. Gehörknöchelchenprothese (10; 20; 30; 50), die zum Ersatz oder Überbrücken mindestens eines Elements der menschlichen Gehörknöchelchenkette ausgebildet ist, mit einem länglichen, Schall übertragenden Prothesenkörper (13; 23; 33; 53), welcher an seinem einen Ende ein erstes Ankoppelelement (11; 21; 31; 51) aufweist, das als eine Kopfplatte (11; 21; 31) zur Anlage der Prothese am Trommelfell oder als ein Clip zur mechanischen Verbindung mit einem Glied der Gehörknöchelchenkette, insbesondere mit dem Ambossfortsatz oder mit dem Hammergriff, oder als ein Anschlussstück (51) zur Schall leitenden Verbindung mit einem Aktor-Endstück (59) eines aktiven Hör-Implantats gestaltet ist, wobei der Prothesenkörper (13; 23; 33; 53) an seinem anderen Ende ein zweites Ankoppelelement (12; 22; 32; 52) zur mechanischen Verbindung mit einem weiteren Glied oder Teilen eines Gliedes der Gehörknöchelchenkette oder direkt mit dem Innenohr aufweist,
**dadurch gekennzeichnet,**
**dass** das erste Ankoppelelement (11; 21; 51) und/oder das zweite Ankoppelelement (32; 52) und/oder der Prothesenkörper eine Markierungseinrichtung aufweist, die mindestens drei voneinander beabstandete, erhabene, an einer Außenfläche des ersten Ankoppelelements (11; 21; 51) und/oder des zweiten Ankoppelelements (32; 52) und/oder des Prothesenkörpers angeordnete Markierungskörper (14; 24; 34"; 54a', 54b', 54") umfasst,
**dass** mindestens drei der erhabenen Markierungskörper (14; 24; 34"; 54") gleichmäßig azimutal um die Längsachse des Prothesenkörpers (13; 23; 33; 53) angeordnet sind, und dass die Markierungskörper (14; 24; 34"; 54a', 54b', 54") so gestaltet sind, dass sie aufgrund ihrer geometrischen Ausgestaltungen und/oder aufgrund ihrer Materialeigenschaften post-operativ in einem im Mittelohr eines Patienten implantierten Zustand der Gehörknöchelchenprothese (10; 20; 30; 50) auf MR (=Magnetresonanz)-Aufnahmen oder auf CT (=Computer-Tomographie)-Aufnahmen getrennt voneinander deutlich erkennbar und eindeutig zuordenbar sind und durch ihre vorbekannten relativen Positionen in oder an der Gehörknöchelchenprothese (10; 20; 30; 50) eine genaue Erkennung und Bestimmung der exakten räumlichen Lage der Gehörknöchelchenprothese (10; 20; 30; 50) im Mittelohr des Patienten ermöglichen.

2. Gehörknöchelchenprothese nach Anspruch 1, **dadurch gekennzeichnet, dass** mindestens einer der Markierungskörper (14; 24; 34"; 54a', 54b', 54") magnetisches Material enthält.

3. Gehörknöchelchenprothese nach Anspruch 1 oder 2, **dadurch gekennzeichnet, dass** mindestens einer der Markierungskörper (14; 24; 34"; 54a', 54b', 54") kohlenwasserstoffhaltiges Kunststoffmaterial enthält.

4. Gehörknöchelchenprothese nach einem der vorhergehenden Ansprüche, **dadurch gekennzeichnet, dass** mindestens einer der erhabenen Markierungskörper (14; 24; 34"; 54a') eine rundliche, vorzugsweise eine kugelige Form aufweist.

5. Gehörknöchelchenprothese nach einem der vorhergehenden Ansprüche, **dadurch gekennzeichnet, dass** mindestens einer der erhabenen Markierungskörper (54b', 54") eine spitze und/oder eckige Form, insbesondere Kegel- oder Würfelform aufweist.

6. Gehörknöchelchenprothese nach einem der vorhergehenden Ansprüche, **dadurch gekennzeichnet, dass** das erste Ankoppelelement (11; 21) als runde diskusförmige Kopfplatte zur Anlage am Trommelfell ausgebildet ist, und dass die erhabenen Markierungskörper (14; 24') am Außenumfang der Kopfplatte und/oder an deren dem Trommelfell im implantierten Zustand der Gehörknöchelchen-prothese (10; 20) abgewandten Unterseite positioniert sind.

7. Gehörknöchelchenprothese nach einem der vorhergehenden Ansprüche, **dadurch gekennzeichnet, dass** die Markierungseinrichtung zusätzlich mindestens einen Hohlraum, eine Bohrung oder eine napfartige Einbuchtung (34') am oder im ersten Ankoppelelement (31) und/oder zweiten Ankoppelelement und/oder am Prothesenkörper umfasst.

8. Gehörknöchelchenprothese nach Anspruch 7, **dadurch gekennzeichnet, dass** der Hohlraum (34'), die Bohrung oder die Einbuchtung (34') mit magnetischem Material und/oder mit kohlenwasserstoffhaltigem Kunststoffmaterial eingefasst oder ausgekleidet ist.

9. Gehörknöchelchenprothese nach Anspruch 7 oder 8, **dadurch gekennzeichnet, dass** die Markierungseinrichtung mehrere Hohlräume, Bohrungen oder napfartige Einbuchtungen (34') umfasst, die gleichmäßig azimutal um die Längsachse des Prothesenkörpers (33) angeordnet sind.

10. Gehörknöchelchenprothese nach einem der vorhergehenden Ansprüche, **dadurch gekennzeichnet, dass** der Prothesenkörper (53) mindestens ein Gelenk, insbesondere ein Kugelgelenk (57) aufweist.

11. Verfahren zur post-operativen Erkennung und exakten Bestimmung der räumlichen Lage einer Markierungseinrichtung mit mindestens drei (14; 24; 34"; 54a', 54b', 54") nach einem der vorhergehenden Ansprüche ausgestatteten Gehörknöchelchenprothese (10; 20; 30; 50) im Mittelohr eines Patienten im implantierten Zustand relativ zur Mittelohr-Umgebung der Prothese, **dadurch gekennzeichnet, dass** nach der Implantation der Gehörknöchelchenprothese (10; 20; 30; 50) ins Mittelohr MR-Bilder oder CT-Bilder vom Mittelohr-Bereich des Patienten aufgenommen werden, und dass die exakte relative Lage der Gehörknöchelchenprothese (10; 20; 30; 50) in Bezug auf ihre Umgebung im Mittelohr aufgrund der in den Aufnahmen erkennbaren, eindeutig zuordenbaren geometrischen Ausgestaltung und/oder Materialeigenschaften der Markierungskörper (14; 24; 34"; 54a', 54b', 54") der Markierungseinrichtung sowie aufgrund der vorbekannten relativen Position der Markierungseinrichtung in oder an der Gehörknöchelchenprothese (10; 20; 30; 50) ermittelt wird.

## Claims

1. Auditory ossicle prosthesis (10; 20; 30; 50) which is designed to replace or bridge at least one element of the human auditory ossicular chain, comprising an elongated, sound-transmitting prosthesis body (13; 23; 33; 53) having at one end a first coupling element (11; 21; 31; 51) which is configured as a top plate (11; 21; 31) for contact of the prosthesis with the tympanic membrane or as a clip for mechanical connection to a member of the ossicular chain, in particular to the incus or to the malleus, or as a connecting piece (51) for sound-transmitting connection to an actuator end piece (59) of an active hearing implant, wherein the prosthesis body (13; 23; 33; 53) has at its other end a second coupling element (12; 22; 32; 52) for mechanical connection to a further member or parts of a member of the ossicular chain, or directly to the inner ear, **characterised in that**
the first coupling element (11; 21; 51) and/or the second coupling element (32; 52) and/or the prosthesis body has a marking device which comprises at least three raised marking bodies (14; 24; 34"; 54a', 54b', 54") which are spaced from one another and are arranged on an outer surface of the first coupling element (11; 21; 51) and/or of the second coupling element (32; 52) and/or of the prosthesis body,
**in that** at least three of the raised marking bodies (14; 24; 34"; 54") are arranged in an azimuthally uniform manner around the longitudinal axis of the prosthesis body (13; 23; 33; 53),
and **in that** the marking bodies (14; 24; 34"; 54a', 54b', 54") are designed such that, as a result of their geometrical configurations and/or their material properties, they are clearly recognisable post-operatively, separately from one another, on MR (magnetic resonance) images or on CT (computer tomography) images in an implanted state of the auditory ossicle prosthesis in the middle ear of a patient, can be unambiguously allocated, and make possible, through their previously known relative positions in or on the auditory ossicle prosthesis (10; 20; 30; 50), a precise recognition and determination of the exact spatial position of the auditory ossicle prosthesis (10; 20; 30; 50) in the middle ear of the patient.

2. Auditory ossicle prosthesis according to Claim 1, **characterised in that** at least one of the marking bodies (14; 24; 34"; 54a', 54b', 54") contains magnetic material.

3. Auditory ossicle prosthesis according to Claim 1 or 2, **characterised in that** at least one of the marking bodies (14; 24; 34"; 54a', 54b', 54") includes hydrocarbon-containing synthetic material.

4. Auditory ossicle prosthesis according to any one of the preceding claims, **characterised in that** at least one of the raised marking bodies (14; 24; 34"; 54a') has a rounded, preferably spherical shape.

5. Auditory ossicle prosthesis according to any one of the preceding claims, **characterised in that** at least one of the raised marking bodies (54b', 54") has a pointed and/or angular shape, in particular a conical or cubical shape.

6. Auditory ossicle prosthesis according to any one of the preceding claims, **characterised in that** the first coupling element (11; 21) is configured as a circular discus-shaped top plate for contact with the tympanic membrane, and **in that** the raised marking bodies (14; 24') are positioned on the outer circumference of the top plate and/or on the underside thereof facing away from the tympanic membrane in the implanted state of the auditory ossicle prosthesis (10; 20).

7. Auditory ossicle prosthesis according to any one of the preceding claims, **characterised in that** the marking device additionally includes at least one cavity, a bore or a dish-shaped recess (34') on or in the first coupling element (31) and/or on or in the second coupling element and/or on the prosthesis body.

8. Auditory ossicle prosthesis according to Claim 7, **characterised in that** the cavity (34'), the bore or the recess (34') is bordered or coated with magnetic material and/or with hydrocarbon-containing synthetic material.

9. Auditory ossicle prosthesis according to Claim 7 or 8, **characterised in that** the marking device includes a plurality of cavities, bores or dish-shaped recesses (34') which are arranged in an azimuthally uniform manner around the longitudinal axis of the prosthesis body (33).

10. Auditory ossicle prosthesis according to any one of the preceding claims, **characterised in that** the prosthesis body (53) includes at least one joint, in particular a ball-and-socket joint (57).

11. Method for the post-operative recognition and precise determination of the spatial position of an auditory ossicle prosthesis (10; 20; 30; 50), equipped with a marking device having at least three marking bodies (14; 24; 34"; 54a', 54b', 54") according to any one of the preceding claims, in the implanted state in the middle ear of a patient, relative to the middle-ear environment of the prosthesis, **characterised in that** after the implantation of the auditory ossicle prosthesis (10; 20; 30; 50) in the middle ear, MR images or CT images of the middle-ear region of the patient are acquired, and **in that** the exact relative position of the auditory ossicle prosthesis (10; 20; 30; 50) in relation to its environment in the middle ear is determined on the basis of the geometrical configuration and/or the material properties of the marking bodies (14; 24; 34"; 54a', 54b', 54") of the marking device, which geometrical configuration and/or material properties can be recognised and unambiguously allocated in the images, and on the basis of the previously known relative position of the marking device in or on the auditory ossicle prosthesis (10; 20; 30; 50).

## Revendications

1. Prothèse d'osselets auditifs (10 ; 20 ; 30 ; 50) qui est réalisée pour le remplacement ou le pontage d'au moins un élément de la chaîne d'osselets auditifs humains, comprenant un corps de prothèse allongé (13 ; 23 ; 33 ; 53) transmettant les sons, qui comporte à l'une de ses extrémités un premier élément de couplage (11 ; 21 ; 31 ; 51) qui est conçu sous la forme d'une plaque de tête (11 ; 21 ; 31) pour l'application de la prothèse contre le tympan ou sous la forme d'une pince pour la liaison mécanique avec un membre de la chaîne d'osselets auditifs, en particulier avec le prolongement l'enclume ou avec le marteau, ou encore sous la forme d'une pièce de raccordement (51) en vue d'une liaison capable de mener les sons avec une pièce terminale d'acteur (59) d'un implant auditif actif, dans laquelle le corps de prothèse (13 ; 23 ; 33 ; 53) comprend à son autre extrémité un second élément de couplage (12 ; 22 ; 32 ; 52) pour la liaison mécanique avec un autre membre ou avec d'autres parties d'un membre de la chaîne d'osselets auditifs ou encore directement avec l'oreille interne,
**caractérisée en ce que** le premier élément de couplage (11 ; 21 ; 51) et/ou le second élément de couplage (32 ; 52) et/ou le corps de prothèse comprend un moyen de marquage, qui inclut au moins trois corps de marquage (14 ; 24 ; 34" ; 54a', 54b', 54") en relief, écartés les uns des autres, agencés sur une surface extérieure du premier élément de couplage (11 ; 21 ; 51) et/ou du second élément de couplage (32 ; 52) et/ou du corps de prothèse,
**en ce qu'**au moins trois des corps de marquage en relief (14 ; 24 ; 34" ; 54") sont agencés régulièrement en situation azimutale autour de l'axe longitudinal du corps de prothèse (13 ; 23 ; 33 ; 53),
et **en ce que** les corps de marquage (14 ; 24 ; 34" ; 54a', 54b', 54") sont ainsi conçus que, en raison de leur conception géométrique et/ou en raison des propriétés de leur matériau, ils sont nettement reconnaissables séparément les uns des autres de façon postopératoire dans un état implanté dans l'oreille moyenne d'un patient, de la prothèse d'osselets auditifs (10 ; 20 ; 30 ; 50) sur des prises de vue en magnéto-résonance (MR) ou des prises de vue en tomographie à l'ordinateur (CT) et sont susceptibles d'être associés de manière univoque, et, en raison de leur position relative préalablement connue dans ou sur la prothèse d'osselets auditifs (10 ; 20 ; 30 ; 50) ils permettent une reconnaissance exacte et une détermination de la position exacte dans l'espace de la prothèse d'osselets auditifs (10 ; 20 ; 30 ; 50) dans l'oreille moyenne du patient.

2. Prothèse d'osselets auditifs selon la revendication 1, **caractérisée en ce que** l'un au moins des corps de marquage (14 ; 24 ; 34" ; 54a', 54b', 54") contient un matériau magnétique.

3. Prothèse d'osselets auditifs selon la revendication 1 ou 2, **caractérisée en ce que** l'un au moins des corps de marquage (14 ; 24 ; 34" ; 54a', 54b', 54") contient une matière plastique qui contient des hydrocarbures.

4. Prothèse d'osselets auditifs selon l'une des revendications précédentes, **caractérisée en ce que** l'un au moins des corps de marquage en relief (14 ; 24 ; 34" ; 54a') présente une forme arrondie, de préférence une forme sphérique.

5. Prothèse d'osselets auditifs selon l'une des revendications précédentes, **caractérisée en ce que** l'un au moins des corps de marquage en relief (54b', 54") présente une forme pointue et/ou une forme avec des coins, en particulier la forme d'un cône ou d'un cube.

6. Prothèse d'osselets auditifs selon l'une des revendications précédentes, **caractérisée en ce que** le premier élément de couplage (11 ; 21) est réalisé sous la forme d'une plaque de tête ronde en forme de disque pour l'application sur le tympan, et **en ce que** les corps de marquage en relief (14 ; 24') sont positionnés à la périphérie extérieure de la plaque de tête et/ou à sa face inférieure, détournée du tympan dans l'état implanté de la prothèse d'osselets auditifs (10 ; 20).

7. Prothèse d'osselets auditifs selon l'une des revendications précédentes, **caractérisée en ce que** le dispositif de marquage inclut additionnellement au moins une cavité, un perçage ou un creux en forme de godet (34') sur ou dans le premier élément de couplage (31) et/ou le second élément de couplage et/ou sur le corps de prothèse.

8. Prothèse d'osselets auditifs selon la revendication 7, **caractérisée en ce que** la cavité (34'), le perçage ou le creux (34') et enserré ou revêtu avec un matériau magnétique et/ou avec une matière plastique contenant des hydrocarbures.

9. Prothèse d'osselets auditifs selon la revendication 7 ou 8, **caractérisée en ce que** le dispositif de marquage inclut plusieurs cavités, perçages ou creux en forme de godet (34'), qui sont agencés régulièrement en situation azimutale autour de l'axe longitudinal du corps de prothèse (33).

10. Prothèse d'osselets auditifs selon l'une des revendications précédentes, **caractérisée en ce que** le corps de prothèse (53) comprend au moins une articulation, en particulier une articulation à rotule (57).

11. Procédé pour la reconnaissance et la détermination exacte postopératoire de la position dans l'espace d'un dispositif de marquage avec au moins trois corps prothèse d'osselets auditifs (10 ; 20 ; 30 ; 50) équipés selon l'une des revendications précédentes dans l'oreille moyenne d'un patient, dans l'état implanté par rapport à l'environnement de l'oreille moyenne de la prothèse, **caractérisé en ce que**, après implantation de la prothèse d'osselets auditifs (10 ; 20 ; 30 ; 50) dans l'oreille moyenne, on prend des images MR ou des images CT de la zone de l'oreille moyenne du patient, et **en ce que** l'on détermine la position relative exacte de la prothèse d'osselets auditifs (10 ; 20 ; 30 ; 50) par référence à son environnement dans l'oreille moyenne grâce à la conception géométrique reconnaissable dans les images et susceptibles d'être associée de manière univoque et/ou grâce aux propriétés des matériaux des corps de marquage (14 ; 24 ; 34" ; 54a', 54b', 54") du dispositif de marquage ainsi que grâce à la position relative préalablement connue du dispositif de marquage dans ou sur la prothèse d'osselets auditifs (10 ; 20 ; 30 ; 50).
